Europäisches Patentamt

(19) European Patent Office (11) Publication number: **0 182 811**

Office européen des brevets **B1**

(12) **EUROPEAN PATENT SPECIFICATION**

(45) Date of publication of the patent specification: 31.05.89

(51) Int. Cl.⁴: **C 07 C 2/00**, C 07 C 5/00

(21) Application number: **85902268.3**

(22) Date of filling: **11.04.85**

(86) International application number: **PCT/US 85/00651**

(87) International publication number: **WO 85/04867 (07.11.85 Gazette 85/24)**

(54) **Process for converting methane to higher hydrocarbon products.**

(30) Priority: 16.04.84 US 601139
16.04.84 US 600657

(43) Date of publication of application: 04.06.86 Bulletin 86/23

(45) Publication of the grant of the patent: 31.05.89 Bulletin 89/22

(84) Designated Contracting States: BE DE FR NL

(56) References cited:
US-A-4 450 311
US-A-4 451 685

JOURNAL OF THE CHINESE CHEMICAL SOCIETY (1981) FANG ET AL, CATALYTIC PYROLYSIS OF METHANE PAGES 265-273

(73) Proprietor: ATLANTIC RICHFIELD COMPANY, 515 South Flower Street, Los Angeles California 90071 (US)

(72) Inventor: MAFFIA, Gennaro, J., 414 Hawthorne Lane, Wallingford, PA 19066 (US)
Inventor: JONES, Andrew, C., 24 Clearbrook Road, Newtown Square, PA 19073 (US)
Inventor: LEONARD, John, J., 31 Fairview Road, Springfield, PA 19064 (US)
Inventor: SOFRANKO, John, A., Box 197 Line Road, R. D. 4, Malvern, PA 19355 (US)

(74) Representative: Cropp, John Anthony David, MATHYS & SQUIRE 10 Fleet Street, London, EC4Y 1AY (GB)

## Description

This invention relates to the conversion of methane to higher hydrocarbons. This invention more particularly relates to the conversion of normally gaseous alkanes into normally liquid hydrocarbons. This invention is especially concerned with the conversion of natural gas to higher hydrocarbons, preferably normally liquid hydrocarbons.

The composition of natural gas at the wellhead varies. For example, the methane content of natural gas may vary from about 40 to about 95 vol.-%. Other constituents of natural gas include ethane, propane, butanes, pentanes (and heavier hydrocarbons), hydrogen sulfide, carbon dioxide, helium and nitrogen.

Natural gases are classified as dry or wet depending on the amount of condensable hydrocarbons contained in it. Condensable hydrocarbons generally comprise $C_3+$ alkanes although some ethane may also be included. Gas conditioning is conventionally employed to alter the composition of wellhead gas, processing facilities usually being located in or near production fields. Conventional processing of wellhead natural gas yields processed natural gas containing at least a major amount of methane that also yield natural gas liquids containing, predominantly, $C_3+$ alkanes.

Considerable attention has been devoted to finding economic means for producing synthetic fuels from natural gas or other feedstock composed of lower alkanes. One approach which has been extensively studied comprises converting natural gas to synthesis gas (mixtures comprising CO and $H_2$) which is then either converted directly to higher hydrocarbons (broadly referred to as a "Fischer-Tropsch" conversion) or first converted to methanol which is subsequently converted to higher hydrocarbons (e.g., by use of the Mobil methanol-to-gasoline processes). A substantial difficulty with both of these synthesis gas-based methods is the formation of oxygenates, which complicates further processing, of liquid products using conventional petroleum refining techniques. As demonstrated by the Sasol plants in South Africa such further processing requires customized refining techniques, particularly adapted to the unique feedstock produced. A process capable of converting methane and/or lower alkanes to hydrocarbons compatible with conventional refinery feedstock would therefore be of substantial value to the industry.

Large-scale use of natural gas often requires a sophisticated and extensive pipeline system. Liquefication has also been employed as a transportation means, but processes for liquifying, transporting and revaporizing natural gas are complex and energy-intensive and require extensive safety precautions. Transport of natural gas has been a continuing problem in the exploitation of natural gas resources. It would be extremely valuable to be able to convert natural gas to more readily handleable or transportable products. Moreover, improved methods for the conversion of natural gas would be of value to the chemical industry.

This invention provides a method for converting a methane-containing gas, particularly natural gas, especially wet natural gas, to higher hydrocarbon, especially normally liquid hydrocarbons. It also provides a method for converting methane to higher hydrocarbon products which are compatible with conventional petroleum refinery feedstocks for the purpose of further processing to more valuable hydrocarbon products. The invention further provides a method for converting natural gas to more easily transportable products. Furthermore it provides an improved method for converting natural gas to higher hydrocarbon products wherein the yield of normally liquid hydrocarbons is enhanced.

Other aspects, objects and the several advantages of the invention will become apparent to those skilled in the art upon reading this disclosure and the appended claims.

## Summary of the invention

In accordance with the present invention there is provided a method for converting methane to higher hydrocarbon products which comprises:

(a) contacting a gas comprising methane with a contact solid at conditions effective to produce higher hydrocarbon products, including $C_2+$ olefins, and water, said contact solid comprising at least one reducible oxide (as herein defined) of at least one metal which oxide when contacted with methane at said contacting conditions is reduced and produces higher hydrocarbon products and water; and

(b) oligomerising at least a portion of said higher hydrocarbon products to still higher hydrocarbon products in the presence of an effective amount of an agent capable of promoting said oligomerisation.

Preferably, the contacting of step (a) is conducted at a temperature selected within the range of 500 to 1000° C and in the substantial absence of catalytically effective Ni, Ah, Pd, Ag, Os, Ir, Pt, Au and compounds thereof to produce higher hydrocarbon products, for example, ethylene which ethylene is oligomerised in step (b).

In a preferred embodiment of the invention (i) the gas comprising methane is provided by the first fraction derived by separating a mixture comprising lower alkanes to form a first fraction enriched in methane relative to said mixture and at least one second fraction enriched in $C_2+$ alkanes relative to said mixture and (ii) said second fraction is dehydrogenated to form a dehydrogenation effluent comprising olefins which $C_2+$ olefins are thereafter oligomerised to form higher hydrocarbon products. At least one fraction comprising $C_2+$ alkanes may be

separated from said higher hydrocarbon products of step (a) or from process streams derived from said dehydrogenation effluent and said higher hydrocarbon products of step (a), and said at least one fraction may be then dehydrogenated to form stream comprising $C_2+$ olefins.

**Detailed description of the invention**

Broadly, any feedstock comprising methane may be employed in the process of this invention. Preferred feedstocks are derived from natural gas or processed natural gas, but this invention is not limited thereto. A more particularly preferred feedstock is wet natural gas. The feedstock may contain other organic or inorganic constituents. Typically the lower alkanes will consist of hydrocarbons containing from 1 to 5 carbon atoms per molecule.

Step (i) of the preferred embodiment of the method of this invention comprises separating a mixture comprising lower alkanes to form a first fraction containing predominately methane and at least one second fraction containing predominately $C_2+$ alkanes. Various methods and means for effecting the separation are well-known in the art. For example, the various techniques for separating condensable hydrocarbons from natural gas may be employed. Most such techniques effect a rough separation of natural gas to form: (1) a gas fraction which is predominately methane but also contains significant amounts of ethane and (2) a natural gas liquids fraction (composed of $C_3+$ alkanes with varying amounts of ethane). Also included within the scope of the separation of this invention are methods which effect a more complete separation of methane from higher alkanes. Such more complete separation techniques are currently preferred for use in the process of this invention.

Also included in aspects of the present invention particularly concerned with producing more readily transportable material from natural gas is the removal of higher alkanes (e.g., in the $C_5+$ range) from the $C_2+$ fraction prior to the subsequent dehydrogenation step (ii). Such separations are included within the broader scope of this invention.

Step (ii) of the preferred embodiment of the method of this invention comprises dehydrogenating the fraction(s) containing predominately $C_2+$ alkanes, separated in step (i), to produce $C_2+$ olefins. It will be apparent to one skilled in the art that separate dehydrogenation of different $C_2$ alkane cuts may sometimes be desirable. However, in a currently preferred embodiment of the process of this invention, a single $C_2+$ alkane fraction is separated in the first step and dehydrogenated to form $C_2+$ olefins.

The dehydrogenation technique employed is not narrowly critical to this invention. Thus, such techniques as thermal dehydrogenation (i.e., pyrolysis or steam cracking), catalytic dehydrogenation (e.g., dehydrogenation over catalysts such as chromia-alumina, calcium nickel phosphate stabilized chromium oxide, etc.), and oxidative dehydrogenation (e.g., dehydrogenation over solids such as bismuthmolybdate, Mg-Cr-ferrites, Mn-ferrites, and other metal oxides and salts to produce dehydrogenated products and coproduct water) may be employed.

The presently preferred method of dehydrogenation is oxidative dehydrogenation. Presently preferred oxidative dehydrogenation solids (or catalysts) include those solids, described below, which may be employed for the conversion of methane to higher hydrocarbons.

Step (a) of the method of this invention comprises contacting a gas comprising methane, e.g. the fraction containing predominately methane separated in step (i) of the preferred embodiment, with a contact solid to form higher hydrocarbon products e g. $C_2+$ olefins.

The methane content of feedstock to the methane conversion zone of the method of the invention may be within the range of about 40 to 100 vol.-%, preferably within the range of about 80 to 100 vol.-%. more preferably within the range of about 90 to 100 vol.-%. In the preferred embodiment of this invention involving steps (i) and (ii) and comprising alkane separation, $C_2+$ alkane dehydrogenation, methane conversion, and olefin oligomerisation, the feedstock will contain predominately methane.

The contact solid which is contacted with methane in step (a) of the present process contains at least one reducible oxide of at least one metal. The term "reducible" identifies those oxides of metals which are reduced by the methane contact. The term "oxide(s) of metal(s)", as used herein, means: (1) one or more metal oxides (i.e. compounds described by the general formula $M_xO_y$ wherein M is a metal and the subscripts x and y designate the relative atomic proportions of metal and oxygen in the composition) and/or (2) one or more oxygen-containing metal compounds, provided that such oxides and compounds have the capability of performing to produce higher hydrocarbon products as set forth herein.

Effective contact solids for the conversion of methane to higher hydrocarbons have previously been found to comprise reducible oxides of metals selected from manganese, tin, indium, germanium, antimony, lead, bismuth and mixtures thereof. See commonly-assigned U.S. Patent Nos. 4 443 649; 4 444 984; 4 443 648; 4 443 645; 4 443 647; 4 443 644; and 4 443 646.

Alkali and alkaline earth metals and compounds thereof have been found to improve the hydrocarbon product selectivity of these solids. The further incorporation of phosphorus into solids promoted by alkali or alkaline earth components enchances catalyst stability. See U.S. Patent Nos. 4 499 322 and 4 495 374.

Reducible oxides of cerium, praseodymium, and terbium have also been found to be effective for the conversion of methane to higher hydrocarbons, especially when the rare earth component is associated with an alkali or alkaline earth metal component. See concurrently filed European patent application 8 590 2267.5 (published as EPA-0 179 131).

The same application discloses and claims a process for the conversion of methane to higher hydrocarbons which comprises contacting methane with a contact solid comprising a reducible oxide of iron or ruthenium and at least one member of the group consisting of alkali metals, alkaline earth metals, and compounds thereof.

The metal components may be associated with other support materials such as silica, magnesia, alumina, titania, zirconia and the like and combinations thereof. When employing solids containing rare earth components-oxides of Ce, Pr and Tb - the rare earth oxides preferably serve as supports.

Reducible oxides of manganese have been found to be particularly desirable for methane conversion, especially when associated with an alkali metal component (preferably sodium). Especially preferred solids comprise silica- and/or magnesia- supported solids containing oxides of manganese and sodium.

The solids contacted with methane in step (a) of the present invention can be prepared by any suitable method, conventional methods such as precipitation, coprecipitation, impregnation or dry mixing can be used. Supported solids may be prepared by methods such as adsorption, impregnation, precipitation, coprecipitation, and dry mixing. When phosphorus is incorporated into the solid, it is desirable to provide it in the form of a phosphate of an alkali or alkaline earth metal.

A suitable method of preparation is to impregnate a support with solutions of the desired metals. Suitable compounds for impregnation include the acetates, acetylacetonates, oxides, carbides, carbonates, hydroxides, sulfides, tartrates, fluorides, chlorides, bromides, or iodides. After impregnation the preparation is dried to remove solvent and the dried solids calcined, preferably in air, at a temperature within the range of about 300 to 1200° C. Particular calcination temperatures will vary depending upon the particular metal compound or compounds employed.

Regardless of how the components of the solids are combined, the composite will be dried and calcined at elevated temperatures prior to use in the process of this invention.

Preferably, methane is contacted with the solid in the substantial absence of catalytically effective nickel, noble metals and compounds thereof (i.e., nickel, rhodium, palladium, silver, osmium, iridium, platinum, and gold) to minimize the deleterious catalytic effects thereof. These metals, when contacted with methane at the temperatures employed in the step (a) of the present invention, tend to promote coke formation, and the metal oxides tend to promote the formation of combustion products rather than the desired hydrocarbons. The term "catalytically effective" is used herein to identify that quantity of one or more nickel and the noble metals and compounds thereof which substantially changes the distribution of products obtained in the step (a) of this invention relative to such contacting in the absence of such metals and compounds thereof.

Operating temperatures for the step (a) of the method of the invention are generally within the range of about 500 to 1000° C. If reducible oxides of metals such as In, Ge or Bi are present in the solid, the particular temperature selected may depend, in part, on the particular reducible metal oxide(s) employed. Thus, reducible oxides of certain metals may require operating temperatures below the upper part of the recited range to minimize sublimation or volatilization of the metals (or compounds) during methane contact. Examples are: reducible oxides of indium, (operating temperatures will preferably not exceed about 850° C) (2) reducible oxides of germanium (operating temperatures will not exceed about 850° C) and (3) reducible oxides of bismuth (operating temperatures will not exceed about 850° C).

Operating pressures for the methane contacting step are not critical to the presently claimed invention. However, general system pressure and partial pressure of methane have been found to affect overall results. Preferred operating pressures are within the range of about 1 to 30 atmospheres.

Effecting the contact under elevated pressure may produce greater amounts of $C_3+$ hydrocarbon products.

Contacting methane and a reducible metal oxide to form higher hydrocarbons from methane also produces a reducible metal oxide and co-product water. The exact nature of the reduced oxides are unknown, and so they are referred to herein as "reduced metal oxides". Regeneration of a reducible metal oxide is readily accomplished by contacting such reduced materials with oxygen (e.g., an oxygen-containing gas such as air) at elevated temperatures, preferably at a temperature selected within the range of about 300 to 1200° C. The particular temperature selected depends upon the metal(s) included in the solid.

In carrying out step (a) of the present process, a single reactor apparatus containing fixed beds of solids may be used with intermittent or pulsed flow of a first gas comprising methane and second gas comprising oxygen (e. g. oxygen, oxygen diluted with an inert gas or air, preferably air). The methane contacting step and the oxygen contacting step may also be performed in physically separate zones with solids recirculating between the two zones.

Thus, one suitable method for synthesizing hydrocarbons from a methane source comprises: (a) contacting a gas comprising methane and particles comprising at least one reducible oxide of at least one metal in a first zone to form higher hydrocarbon products, co-product water, and reduced metal oxide; (b) removing particles comprising reduced metal oxide from the first zone and contacting the reduced particles in a second zone with an oxygen-containing gas to form particles comprising a reducible metal oxide; and (c) returning the particles produced in the second zone to the first zone. The steps are preferably repeated at least periodically, and more preferably the steps are continuous. In one more preferred embodiment solids are continuously circulated between at least one methane contact zone and at least one oxygen contact zone.

Particles comprising a reducible metal oxide which are contacted with methane may be maintained as fluidized, ebullating, or entrained beds of solids. Preferably methane is contacted with a fluidized bed of solids.

Similarly, particles comprising reduced metal oxide which are contacted with oxygen may be maintained as fluidized, ebullating or entrained beds of solids. Preferably oxygen is contacted with a fluidized bed of solids.

In one more preferred embodiment of the present invention, methane feedstock and particles comprising promoted oxidative synthesizing agent are continuously introduced into a methane contact zone maintained at synthesizing conditions. Synthesizing conditions include the temperatures and pressures described above. Gaseous reaction products from the methane contact zone (separated from entrained solid) may be further processed - e.g., they may be passed through a fractionating system wherein the desired hydrocarbon products are separated from unconverted methane and combustion products. Unconverted methane may be recovered and recycled to the methane contact zone.

In this embodiment, particles comprising reduced metal oxide are contacted with oxygen in an oxygen contact zone for a time sufficient to oxidize at least a portion of the reduced oxide to produce a reducible metal oxide and to remove, i.e., combust, at least a portion of any carbonaceous deposit which may form on the particles in the methane contact zone. The conditions of the oxygen contact zone will preferably include a temperature selected within the range of about 300 to 1200°C, pressures of up to about 30 atmospheres, and average particle contact time within the range of about 1 to 120 minutes. Sufficient oxygen is preferably provided to oxidize all reduced metal oxide to produce a reducible oxide and to completely combust any carbonaceous material deposited on the particles. At least a portion of the particles comprising promoted oxidative synthesizing agent which are produced in the oxygen contact zone are returned to the methane contact zone.

The rate of solids withdrawal from the methane contact zone is desirably balanced with the rate of solids passing from the oxygen contact zone to the methane contact zone so as to maintain a substantially constant inventory of particles in the methane contact zone, thereby enabling steady state operation of the synthesizing system.

The effluent produced by the step (a) of the method of this invention comprises unconverted methane and higher hydrocarbons (especially ethane and ethylene), as well as carbon oxides and water. It is within the scope of the present invention to recover a portion of this effluent (e.g., methane) for recycle to the methane contact zone. Similarly, carbon oxides and water may be removed from the effluent prior to further treatment of the effluent in accordance with the present invention.

Whether or not such intermediate separations are employed, a gas stream comprising $C_2+$ olefins is recovered from the effluent of step (a) and is passed to step (b) of the process of this invention wherein olefins are oligomerized to produce higher hydrocarbon products.

Step (b) of the method of this invention comprises oligomerising the $C_2+$ olefins present in the effluent of step (a) to form higher hydrocarbon products. The oligomerization techniques employed are not narrowly critical to this invention.

Numerous catalysts and processes are known for the oligomerization of olefins generally, and of ethylene particularly. For example, phosphoric acid supported on a kieselguhr base has been widely used for making polymer gasoline (i. e. olefinic hydrocarbon liquids within the gasoline boiling range) from refinery gases. Other catalysts which have been employed for similar purposes include the oxides of cobalt, nickel, chromium, molybdenum and tungsten on supports such as alumina, silica-alumina, kieselguhr, carbon and the like.

Included within the broad scope of the present invention are all catalysts and processes which are effective for the oligomerization of olefins to higher hydrocarbons, preferably olefinic hydrocarbon liquids within the gasoline boiling range. Without intending to limit the scope of the claimed invention, most oligomerization catalysts may be classified in one of two general categories: metal catalysts and acid catalysts. They may also be classified as heterogeneous (solid) catalysts or homogeneous (liquid-phase) catalysts.

For examples of metal catalysts based on nickel, see U.S. Patent Nos. 2 828 347; 3 459 826; 3 527 839; 3 954 668; 3 959 400; 4 260 844; 4 272 406; 4 288 648; 4 293 725; and Industrial Chemistry, 47 pp. 752, et seq. (1955). Note that these catalysts require a donor ligand and a Lewis acid. For examples of metal catalysts based on palladium, see U.S. Patent Nos. 3 644 565; 3 728 415; 3 738 977; 3 758 626; and 3 920 763. An example of metal catalysts based on chromium is found in U.S. Patent No. 3 709 954. An example of metal catalysts based on cobalt is found in Industrial and Engineering Chemistry, 42, pp. 2580, et seq. (1950). Examples of metal catalysts based on titanium found in U.S. Patent No. 3 981 941 and 4 110 410. An example of metal catalysts based on tungsten is found in U.S. Patent No. 3 903 193. An example of metal catalysts based on rhenium is found in U.S. Patent No. 3 393 251.

Examples of phosphoric acid catalyst are described in U.S. Patent Nos. 2 383 318 and 3 887 634 and also in Industrial and Engineering Chemistry, 27, pp. 1364, et seq. (1935). Acid catalysts based on chlorided or fluorided alumina are found in U.S. Patent Nos. 3 364 191 and 3 515 769 and also in USSR Patent No. 107 176.

Other acid catalysts of particular interest in the context of the present invention are siliceous, crystalline molecular sieves. Such silica-containing crystalline materials include materials which contain, in addition to silica, significant amounts of alumina. These crystalline materials are frequently named "zeolites", i.e., crystalline aluminosilicates. Silica-containing crystalline materials also include essentially aluminum-free silicates. These crystalline materials are exemplified by crystalline silica polymorphs (e. g. silicalite, disclosed in U.S. Patent No. 4 061 724 and organosilicates, disclosed in U.S. Patent No. RE. 29948), chromiasilicates (e.g., CZM), ferrosilicates and galliosilicates (see U.S. Patent No. 4 238 318), and borosilicates (see U.S. Patent Nos. 4 226 420; 4 269 813; and 4 327 236).

Crystalline aluminosilicate zeolites are best exemplified by ZSM-5 (see U.S. Patent Nos. 3 702 886 and 3 770 614), ZSM-11 (see U.S. Patent No. 3 709 979), ZSM-12 (see U.S. Patent No. 3 832 449), ZSM-21 and ZSM-38 (see U.S. Patent No. 3 948 758), ZSM-23 (see U.S. Patent No. 4 076 842), and ZSM-35 (see U.S. Patent No. 4 016 246). Examples of processes for the conversion of low molecular weight olefins over zeolites are found in U.S. Patent Nos. 2 972 643; 3 325 465; 3 960 978; 3 972 832; 4 021 502; 4 044 065; 4 150 062; and 4 254 295. Also see U.S. Patent Nos. 4 417 086 and 4 417 087 wherein oligomerization processes employing fluidized crystalline molecular sieves are disclosed.

Metal oligomerization catalysts in general are more sensitive to feed impurities, (e.g., water, carbon monoxide, dienes, etc.) than are the acid catalysts. Although homogeneous, metal catalysts are quite active, the need for dry feeds, solvents, and other measures to prevent catalyst deactivation and precipitation is disadvantageous and suggests an obvious advantage to supported, heterogeneous, metal catalyst. Homogeneous acid catalysts are effective but are also corrosive and tend to form two liquid-phase systems with the non-polar hydrocarbon oligomerization products. Considering the foregoing observations, heterogeneous acid catalysts are the preferred catalyst for use in the oligomerization step of the present invention. Of the heterogeneous acid catalysts, acid zeolites are especially preferred, particularly zeolites of the ZSM-type and borosilicates.

The oligomerisation step of the present invention may be performed according to any of the numerous processes known to those skilled in the art.

In the preferred embodiment of this invention wherein (i) the gas comprising methane is provided by the first fraction derived by separating a mixture comprising lower alkanes to form a first fraction enriched in methane relative to said mixture and at least one second fraction enriched in $C_2+$ alkanes relative to said mixture and (ii) said second fraction is dehydrogenated to form a dehydrogenation effluent comprising $C_2+$ olefins which $C_2+$ olefins are thereafter oligomerised to form higher hydrocarbon products, and the effluents from the dehydrogenation and methane-contacting steps are combined, the combined stream will typically comprise a mixture of olefins containing a major amount of diluent.

It is also within the scope of the present invention to oligomerise the $C_2+$ olefins contained in the dehydrogenation effluent and the effluent step of (a) separately. However, one principal advantage of the above-mentioned preferred embodiment of this invention is the enhanced efficiencies realised when the effluents are combined and oligomerised.

Also within the scope of the present invention are flow schemes wherein the effluents are combined and then fractionated to recover various cuts of $C_2+$ olefins. Such will be apparent to one skilled in the art. Again, however, in one embodiment of this invention, it is not necessary to perform such a fractionation.

In addition to olefins, the effluent of step (a) may contain varying amounts of $C_2+$ alkanes. Moreover, some alkane formation may occur while oligomerising $C_2+$ olefins. Separation of $C_2+$ alkanes from the process streams may occur at various points in a process comprising methane conversion to olefins and olefin oligomerization to higher hydrocarbons. Such separation and recycle is within the scope of the present invention.

However, in a preferred, distinct, embodiment of the present invention, $C_2+$ alkanes recovered from the effluent of step (a) and/or $C_2+$ alkanes recovered from process streams derived from the effluent of step (a) or dehydrogenation effluent of the present invention are recycled to the second, dehydrogenation step of this invention. Such recycle enchances the overall hydrocarbon conversion efficiencies attained by the more general method of this invention.

**Claims**

1. A method for converting methane to higher hydrocarbon products characterised in that said method comprises:

(a) contacting a gas comprising methane with a contact solid at conditions effective to produce higher hydrocarbon products, including $C_2+$ olefins, and water, said contact solid comprising at least one reducible oxygen-containing compound of at least one metal which compound when contacted with methane at said contacting conditions is reduced and produces higher hydrocarbon products and water; and

(b) oligomerising at least a portion of said higher hydrocarbon products to still higher hydrocarbon products in the presence of an effective amount of an agent capable of promoting said oligomerisation.

2. A method as claimed in claim 1 wherein the contacting of step (a) is conducted at a temperature selected within the range of 500 to 1000° and in the absence of catalytically effective Ni, Rh, Pd, Ag, Os, Ir, Pt, Au and compounds thereof.

3. A method as claimed in claim 1 or claim 2 wherein said higher hydrocarbon products produced in step (a) comprise ethylene and wherein ethylene is oligomerised in step (b).

4. A method as claimed in any one of claims 1 to 3 wherein

(i) the gas comprising methane in step (a) is provided by the first fraction derived by separating a mixture comprising lower alkanes to form a first fraction enriched in methane relative to said mixture and at least one second fraction enriched in $C_2+$ alkanes relative to said mixture; and

(ii) said second fraction is dehydrogenated to form a dehydrogenation effluent comprising $C_2+$ olefins which $C_2+$ olefins are thereafter oligomerised to form higher hydrocarbon products.

5. A method as claimed in claim 4 wherein $C_2+$ olefin-containing effluent of step (a) and $C_2+$ olefin-containing dehydrogenation effluent of step (ii) are combined and the combined material is oligomerised to form higher hydrocarbon products.

6. A method as claimed in claim 4 or claim 5 wherein at least one fraction comprising $C_2+$ alkanes is separated from said higher hydrocarbon products of step (a) or from process streams derived from said dehydrogenation effluent and said higher hydrocarbon products of step (a), and said at least one fraction is dehydrogenated to form a stream comprising $C_2+$ olefins.

7. A method as claimed in claim 6 wherein said at least one fraction containing $C_2+$ alkanes is recycled to step (ii) for dehydrogenation.

8. The method as claimed in any one of claims 1 to 7 wherein the gas comprising methane contains 40 to 100 vol.-% methane, preferably 80 to 100 vol.-% methane, more preferably 90 to 100 vol.-% methane.

9. A method as claimed in any one of claims 1 to 7 wherein the gas comprising methane is derived from natural gas or processed natural gas.

10. A method as claimed in any one of claims 1 to 9 wherein said at least one reducible oxygen-containing compound is associated with a support material.

11. A method as claimed in any one of claims 1 to 10 wherein said at least one reducible oxygen-containing compound is selected from oxides of Mn, Sn, In, Ge, Sb, Pb, Bi and mixtures thereof, and is preferably an oxide of Mn.

12. A method as claimed in any one of claims 1 to 11 wherein the oligomerisation is catalysed by a heterogeneous acid catalyst.

13. A method as claimed in claim 12 wherein said catalyst is a siliceous, crystalline molecular sieve.

14. A method as claimed in claim 13 wherein said molecular sieve is selected from ZSM-type zeolites and borosilicates.

**Patentansprüche**

1. Verfahren zur Umwandlung von Methan in höhere Kohlenwasserstoffprodukte, dadurch gekennzeichnet, daß man:

(a) ein Gas, das Methan enthält, mit einem Kontaktfeststoff bei Bedingungen in Kontakt bringt, die zur Herstellung höherer Kohlenwasserstoffprodukte, einschließlich $C_2+$ Olefine und Wasser wirksam sind, wobei der Kontaktfeststoff mindestens eine reduzierbare sauerstoffhaltige Verbindung von mindestens einem Metall enthält, wobei die Verbindung, wenn mit Methan bei den Kontaktbedingungen in Kontakt gebracht, reduziert wird und höhere Kohlenwasserstoffprodukte und Wasser ergibt; und

(b) mindestens einen Teil der höheren Kohlenwasserstoffprodukte zu noch höheren Kohlenwasserstoffprodukten in Gegenwart einer wirksamen Menge eines die Oligomerisation fördernden Mittels oligomerisiert.

2. Verfahren nach Anspruch 1, bei dem der Kontakt in Stufe (a) bei einer Temperatur, ausgewählt aus dem Bereich von 500 bis 1000° C und in Abwesenheit von katalytisch wirksamen Ni, Rh, Pd, Ag, Os, Ir, Pt, Au und deren Verbindungen durchgeführt wird.

3. Verfahren nach Anspruch 1 oder 2, bei dem die in Stufe (a) erzeugten höheren Kohlenwasserstoffprodukte Ethylen enthalten und bei dem Ethylen in Stufe (b) oligomerisiert wird.

4. Verfahren nach einem der Ansprüche 1 bis 3, bei dem

(i) das Gas, das in Stufe (a) Methan enthält, durch die erste Fraktion, die durch Abtrennen einer Mischung, die niedrigere Alkane enthält, um eine erste Fraktion, angereichert an Methan bezüglich dieser Mischung zu erhalten und mindestens eine zweite Fraktion, angereichert an $C_2+$ Alkanen bezüglich dieser Mischung zur Verfügung gestellt wird; und

(ii) die zweite Fraktion dehydriert wird um einen Dehydrierungsabfluß, der $C_2+$ Olefine enthält, zu bilden, wobei die $C_2+$ Olefine anschließend zur Bildung höherer Kohlenwasserstoffprodukte oligomerisiert werden.

5. Verfahren nach Anspruch 4, bei dem der $C_2+$ olefinhaltige Abfluß aus Stufe (a) und der $C_2+$ olefinhaltige Dehydrierungsabfluß aus Stufe (ii) vereinigt werden und das vereinigte Material zur Bildung höherer Kohlenwasserstoffprodukte oligomerisiert wird.

6. Verfahren nach Anspruch 4 oder 5, bei dem mindestens eine Fraktion, die $C_2+$ Alkane enthält, von den höheren Kohlenwasserstoffprodukten aus Stufe (a) oder von Verfahrensströmen, die von dem Dehydrierungsabfluß und den höheren Kohlenwasserstoffprodukten aus Stufe (a) stammen, abgetrennt wird und wobei zumindest eine Fraktion zur Bildung eines Stromes, der $C_2+$ Olefine enthält, dehydriert wird.

7. Verfahren nach Anspruch 6, bei dem mindestens eine Fraktion, die $C_2+$ Alkane enthält in Stufe (ii) zur Dehydrierung zurückgeführt wird.

8. Verfahren nach einem der Ansprüche 1 bis 7, bei dem das Gas, das Methan enthält, 40 bis 100 Vol.-% Methan, vorzugsweise 80 bis 100 Vol.-% Methan, bevorzugter 90 bis 100 Vol.-% Methan enthält.

9. Verfahren nach einem der Ansprüche 1 bis 7, bei dem das Gas, das Methan enthält, aus Erdgas oder behandeltem Erdgas erhalten wird.

10. Verfahren nach einem der Ansprüche 1 bis 9, bei dem die mindestens eine reduzierbare sauerstoffhaltige Verbindung mit einem Trägermaterial verbunden ist.

11. Verfahren nach einem der Ansprüche 1 bis 10, bei dem die mindestens eine reduzierbare sauerstoffhaltige Verbindung ausgewählt ist aus den Oxiden von Mn, Sn, In, Ge, Sb, Pb, Bi und deren Mischungen und vorzugsweise ein Oxid von Mn ist.

12. Verfahren nach einem der Ansprüche 1 bis 11, bei dem die Oligomerisation durch einen heterogenen Säurekatalysator katalysiert wird.

13. Verfahren nach Anspruch 12, bei dem der Katalysator ein siliciumdioxidhaltiges kristallines Molekularsieb ist.

14. Verfahren nach Anspruch 13, bei dem das Molekularsieb aus Zeolithen vom ZSM-Typ und Borosilicaten ausgewählt wird.

**Revendications**

1. Procédé pour convertir du méthane en produits hydrocarbonés supérieurs, caractérisé en ce qu'il comprend:

(a) la mise en contact d'un gaz comprenant du méthane avec une masse de contact dans des conditions efficaces pour produire des produits hydrocarbonés supérieurs, comprenant des oléfines en $C_2+$, et de l'eau, cette masse de contact comprenant au moins un composé réductible contenant de l'oxygène d'au moins un métal, lequel composé est réduit et produit des hydrocarbures supérieurs et de l'eau lorsqu'il est mis en contact avec du méthane dans ces conditions de contact; et

(b) l'oligomérisation d'au moins une portion de ces produits hydrocarbonés supérieurs en produits hydrocarbonés encore supérieurs en présence d'une quantité efficace d'un agent capable de favoriser cette oligomérisation.

2. Procédé suivant la revendication 1, caractérisé en ce que la mise en contact de l'étape (a) est conduite à une température choisie dans la gamme de 500 à 1000°C et en l'absence de Ni, Rh, Pd, Ag, Os, Ir, Pt, Au et leurs composés ayant une efficacité catalytique.

3. Procédé suivant la revendication 1 ou la revendication 2, caractérisé en ce que les produits hydrocarbonés supérieurs produits dans l'étape (a) comprennent de l'éthylène et que l'éthyléne est oligomérisé dans l'étape (b).

4. Procédé suivant l'une quelconque des revendications 1 à 3, caractérisé en ce que:

(i) le gaz comprenant du méthane dans l'étape (a) est fourni par la première fraction obtenue par séparation d'un mélange comprenant des alcanes inférieurs pour former une première fraction enrichie en méthane par rapport à ce mélange et au moins une seconde fraction enrichie en alcanes en $C_2+$ par rapport à ce mélange; et

(ii) cette seconde fraction est déshydrogénée pour former un effluent de déshydrogénation comprenant des oléfines en $C_2+$ lesquelles oléfines en $C_2+$ sont ensuite oligomérisées pour former des produits hydrocarbonés supérieurs.

5. Procédé suivant la revendication 4, caractérisé en ce que l'effluent de l'étape (a) contenant des oléfines en $C_2+$ et l'effluent de déshydrogénation de l'étape (ii) contenant des oléfines en $C_2+$ sont combinés et, que la matière combinée est oligomérisée pour former des produits hydrocarbonés supérieurs.

6. Procédé suivant la revendication 4 ou la revendication 5, caractérisé en ce qu'au moins une fraction comprenant des alcanes en $C_2+$ est séparée à partir de ces produits hydrogénés supérieurs de l'étape (a) ou des courants de procédé dérivés de cet effluent de déshydrogénation et de ces produits hydrocarbonés supérieurs de l'étape (a), et qu'au moins une desdites fractions est déshydrogénée pour former un courant comprenant des oléfines en $C_2+$.

7. Procédé suivant la revendication 6, caractérisé en ce que cette fraction au moins présente qui contient des alcanes en $C_2+$ est recyclée vers l'étape (ii) pour être déshydrogénée.

8. Procédé suivant l'une quelconque des revendications 1 à 7, caractérisé en ce que le gaz comprenant du méthane contient de 40 à 100 % en volume de méthane, de préférence de 60 à 100 % en volume de méthane et de façon plus avantageuse de 90 à 100 % en volume de méthane.

9. Procédé suivant l'une quelconque des revendications 1 à 7, caractérisé en ce que le gaz comprenant du méthane provient d'un gaz naturel ou d'un gaz naturel traité.

10. Procédé suivant l'une quelconque des revendications 1 à 9, caractérisé en ce que ce composé réductible contenant de l'oxygène au moins présent est associé à un matériau de support.

11. Procédé suivant l'une quelconque des revendications 1 à 10, caractérisé en ce que ce composé réductible contenant de l'oxygène au moins présent est choisi parmi les oxydes de Mn, Sn, In, Ge, Sb, Pb, Bi et leurs mélanges, et est de préférence un oxyde de Mn.

12. Procédé suivant l'une quelconque des revendications 1 à 11, caractérisé en ce que l'oligomérisation est catalysée par un catalyseur acide héterogène.

13. Procédé suivant la revendication 12, caractérisé en ce que ce catalyseur est un tamis moléculaire cristallin, siliceux.

14. Procédé suivant la revendication 13, caractérisé en ce que ce tamis moléculaire est choisi parmi des zéolites de type ZSM et des borosilicates.